# EUROPEAN PATENT APPLICATION

(11) **EP 2 594 195 A1**
(43) Date of publication of application: **22.05.2013**
(21) Application number: 12160916.8
(22) Date of filing: 23.03.2012
(51) Int. Cl.: A61B 5/0245, A61B 5/024

(54) **Heart rate alarm system**

(30) Priority: 21.11.2011 TW 100142515
(71) Applicant: Zoetronics Technology Co., Ltd., Taipei City 104 (TW)
(72) Inventor: Chen, Jen-Ran, 116 Taipei City (TW); Lee, Ren-Guey, 106 Taipei City (TW); Lai, Chien-Chih, 515 Changhua County (TW); Wang, Hsi-Wen, 235 New Taipei City (TW); Chen, Chun-Chang, 404 Taichung City (TW)
(74) Representative: Becker Kurig Straus

(57) **Abstract**

A heart rate alarm system (100, 200) includes an article of clothing (102), at least two conductive units (104, 106), at least one alarm device (108), a heart rate measurement unit (110), a first transmission unit (112), a second transmission unit (114), and an operation unit (116). The at least two conductive units (104, 106) are coupled to each other through at least one conductive fibric (118). The heart rate measurement unit (110) measures a heart rate through the at least two conductive units (104, 106), and generates a control signal and a heart rate value according to the heart rate. The first transmission unit (112) transmits the control signal to the at least one alarm device (108) and transmits the heart rate value. The at least one alarm device (108) generates at least one alarm signal according to the control signal. The operation unit (116) calculates a heart beat period, a heart physiological age, indicators of autonomic nervous system, and/or a calorie consumption indicator.

## Description

### Field of the Invention

The present invention relates to a heart rate alarm system according to the pre-characterizing clause of claim 1.

### Background of the Invention

The heart rate measurement of a user can help a doctor estimate a plurality of indicators of a body of the user (such as a heart beat period , a heart physiological age, indicators of autonomic nervous system, a calorie consumption indicator of the user, and so on). However, a heart rate signal of the user generated by a heart activity of the user is transmitted to different parts of the body through nerve fibers of the body, therefore heart rate signal strengths of different parts of the body are different, resulting in a location for measuring the heart rate signal of the user being one significant issue for design of a heart rate measurement tool.

In the prior art, other than medical equipment, the main heart rate measurement tool is a chest strap attached on the chest of the user. The chest strap can measure a heart rate of the user through two electrodes or three electrodes attached on the chest near the heart of the user (the electrode includes conductive adhesive or conductive fibrics). However, the chest strap may obtain poorer measurement quality of the heart rate due to the chest strap slipping to an improper location after the user sweats. Therefore, the prior art is not a proper choice for the user.

### Summary of the Invention

This in mind, the present invention aims at providing a heart rate alarm system that not only can be beautiful, comfortable and accurate, but can also monitor a heart rate of a user to alarm the user at any time and place.

This is achieved by a heart rate alarm system according to claim 1. The dependent claims pertain to corresponding further developments and improvements.

As will be seen more clearly from the detailed description following below, the claimed heart rate alarm system includes an article of clothing, at least two conductive units, at least one alarm device, a heart rate measurement unit, a first transmission unit, a second transmission unit, and an operation unit. Two conductive units of the at least two conductive units are two elastic bands, and the at least two conductive units are coupled to each other through at least one conductive fibric of the clothing. The at least one alarm device is disposed on the clothing for generating at least one alarm signal. The heart rate measurement unit is coupled to the at least two conductive units for measuring a heart rate of a user to generate a heart rate value of the user through the at least two conductive units, determining whether the heart rate value of the user is normal, and generating a control signal according to a determination result through the at least two conductive units. The first transmission unit is coupled to the heart rate measurement unit for transmitting the control signal to the at least one alarm device, and transmitting the heart rate value. The second transmission unit is used for receiving the heart rate value of the user. The operation unit is coupled to the second transmission unit for calculating a heart beat period, a heart physiological age, indicators of an autonomic nervous system, and/or a calorie consumption indicator of the user according to the heart rate value of the user.

### Brief Description of the Drawings

In the following, the invention is further illustrated by way of example, taking reference to the accompanying drawing thereof:
FIG. 1 is a diagram illustrating a heart rate alarm system according to an embodiment, and
FIG. 2 is a diagram illustrating a heart rate alarm system according to another embodiment.

### Detailed Description

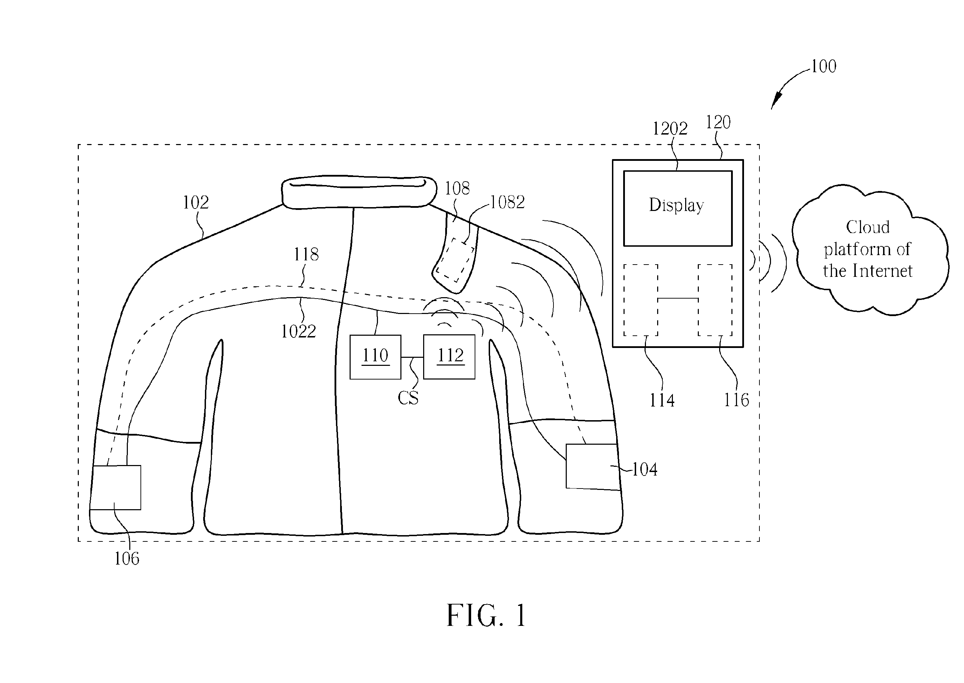

Please refer to FIG. 1. FIG. 1 is a diagram illustrating a heart rate alarm system 100 according to an embodiment. The heart rate alarm system 100 includes an article of clothing 102, two conductive units 104, 106, an alarm device 108, a heart rate measurement unit 110, a first transmission unit 112, a second transmission unit 114, and an operation unit 116. As shown in FIG. 1, the clothing 102 is long-sleeved clothing. However, in another embodiment of the present invention, the clothing 102 is short-sleeved clothing. The two conductive units 104, 106 are two elastic bands including conductive fibrics and/or conductive adhesive, and the two conductive units 104, 106 are connected to two cuffs of the clothing 102 through at least two metal tenons, at least two metal buttons, at least two conductive velcros, and/or at least two magnets. However, the present invention is not limited to the two conductive units. Therefore, the heart rate alarm system 100 can include at least two conductive units. In addition, the present invention is not limited to the two conductive units 104, 106 being connected to the two cuffs of the clothing 102 through the at least two metal tenons, the at least two metal buttons, the at least two conductive velcros, and/or the at least two magnets. Therefore, the two conductive units 104, 106 need only be located at two opposite sides of a heart of a user for the heart rate alarm system 100 to obtain a better heart rate signal of the user. In addition, when the two conductive units 104, 106 are connected to the two cuffs of the clothing 102 through the at least two metal tenons, the at least two metal buttons, the at least two conductive velcros, and/or the at least two magnets, the two conductive units 104, 106 are coupled to each other through a conductive fibric 118 of the clothing 102.However, the present invention is not limited to two conductive units 104, 106 being coupled to each other through the conductive fibric 118 of the clothing 102.Therefore, the two conductive units 104, 106 can be coupled to each other through at least one conductive fibric of the clothing 102. As shown in FIG. 1, the alarm device 108 disposed on the clothing 102 includes a light alarm unit 1082, where the alarm device 108 is used for generating an alarm signal (such as a flash) and the light alarm unit 1082 can be a light-emitting diode alarm unit. However, the present invention is not limited to the alarm device 108 including the light alarm unit 1082. Therefore, the alarm device 108 can include a special sound effect unit, or both a light alarm unit and a special sound effect unit. When the alarm device 108 includes the special sound effect unit, an alarm signal generated by the alarm device 108 is a special sound effect; when the alarm device 108 includes both the light alarm unit and the special sound effect unit, alarm signals generated by the alarm device 108 are a flash and a special sound effect. In addition, the present invention is not limited to the heart rate alarm system 100 only including the alarm device 108. Therefore, the heart rate alarm system 100 can include at least one alarm device. The heart rate measurement unit 110 is coupled to the clothing 102 through at least one metal tenon, at least one metal button, at least one conductive velcro, and/or at least one magnet, and coupled to the two conductive units 104, 106 through a first transmission line 1022 of the clothing 102, where the first transmission line 1022 can be a conductive fibric. The heart rate measurement unit 110 can measure a heart rate of the user through the two conductive units 104, 106 to generate a heart rate value of the user, and determine whether the heart rate value of the user is normal and generate a control signal CS according to a determination result. The first transmission unit 112 is coupled to the heart rate measurement unit 110 through at least one metal tenon, at least one metal button, at least one conductive velcro, and/or at least one magnet for transmitting the control signal CS to the alarm device 108, and transmitting the heart rate value, where the first transmission unit 112 transmits the control signal CS to the alarm device 108 through a Wireless Local Area Network (WLAN), a Zigbee (IEEE 802.15.4), a Bluetooth, a Bluetooth Low Energy (BLE), a Worldwide Interoperability for Microwave Access (WiMAX), a Global System for Mobile Communications (GSM), a General Packet Radio Service (GPRS), a Third Generation (3G), or an Actor Network Theory+ (Ant+) technology. However, in another embodiment of the present invention, the first transmission unit 112 transmits the control signal CS to the alarm device 108 through at least one conductive fibric of the clothing 102. The second transmission unit 114 receives the heart rate value of the user through a wireless transmission manner, where the wireless transmission manner can be the Wireless Local Area Network, the Zigbee, a Bluetooth, a Bluetooth Low Energy, the Worldwide Interoperability for Microwave Access, the Global System for Mobile Communications, the General Packet Radio Service, the Third Generation, or the Actor Network Theory+ technology. However, in another embodiment of the present invention, the second transmission unit 114 communicates with the first transmission unit 112 through a transmission line. Therefore, the second transmission unit 114 receives the heart rate value of the user through the transmission line. The operation unit 116 is coupled to the second transmission unit 114 for calculating a heart rate variation of the user according to the heart rate value received by the second transmission unit 114, and calculating a heart beat period, a heart physiological age, indicators of an autonomic nervous system, and/or a calorie consumption indicator of the user according to the heart rate variation. The second transmission unit 114 and the operation unit 116 are located outside the clothing, and included by a cell phone 120. Therefore, the operation unit 116 can utilize a display 1202 of the cell phone 120 to display the heart beat period, the heart physiological age, the indicators of autonomic nervous system, and/or the calorie consumption indicator of the user. However, the present invention is not limited to the second transmission unit 114 and the operation unit 116 being included in the cell phone 120. Therefore, the second transmission unit 114 and the operation unit 116 can also be included by a smart phone, a tablet computer, a notebook computer, a personal digital assistant or a desktop computer. As shown in FIG. 1, the second transmission unit 114 further uploads the heart beat period, the heart physiological age, the indicators of the autonomic nervous system, and/or the calorie consumption indicator of the user to a medical service cloud platform of the Internet through a wireless transmission manner. Then, the medical service cloud platform can perform long-term recording, analysis, and/or monitoring of the heart beat period, the heart physiological age, the indicators of autonomic nervous system, and/or the calorie consumption indicator of the user. However, in another embodiment of the present invention, the second transmission unit 114 uploads the heart beat period, the heart physiological age, the indicators of autonomic nervous system, and/or the calorie consumption indicator of the user to the medical service cloud platform of the Internet through a transmission line.

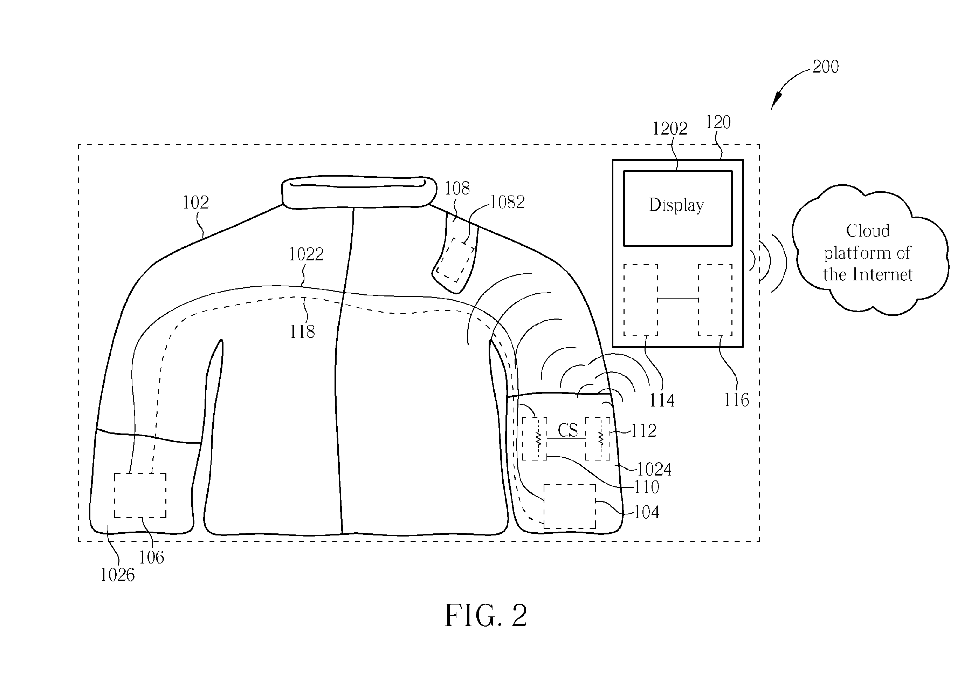

Please refer to FIG. 2. FIG. 2 is a diagram illustrating a heart rate alarm system 200 according to another embodiment. A difference between the heart rate alarm system 200 and the heart rate alarm system 100 is that the clothing 102 further includes two pieces of tarpaulin 1024, 1026. Only the two pieces of tarpaulin 1024, 1026 being located at two opposite sides of the heart of the user falls within the scope of the present invention. The two elastic bands 104, 106 are disposed at inner sides of the two pieces of tarpaulin 1024, 1026, respectively. The heart rate measurement unit 110 and the first transmission unit 112 are disposed at the inner side of the piece of tarpaulin 1024. However, the present invention is not limited to the two pieces of tarpaulin 1024, 1026. Therefore, a number of tarpaulins are equal to a number of conductive units, and the present invention is not limited to the heart rate measurement unit 110 and the first transmission unit 112 being disposed at the inner side of the piece of tarpaulin 1024.Therefore, the heart rate measurement unit 110 and the first transmission unit 112 can be disposed at inner sides of the two pieces of tarpaulin 1024, 1026, respectively. In addition, at least one passive component of the heart rate measurement unit 110 is composed of at least one conductive fibric of the clothing 102. As shown in FIG. 2, the at least one passive component of the heart rate measurement unit 110 is a resistor. However, the present invention is not limited to the at least one passive component of the heart rate measurement unit 110 being a resistor. Therefore, the at least one passive component of the heart rate measurement unit 110 can be a resistor, an inductor, or a capacitor. In addition, at least one passive component of the first transmission unit 112 can also be composed of at least one conductive fibric of the clothing 102. As shown in FIG. 2, the at least one passive component of the first transmission unit 112 is a resistor. But, the present invention is not limited to the at least one passive component of the first transmission unit 112 being a resistor. Therefore, the at least one passive component of the first transmission unit 112 can be a resistor, an inductor, or a capacitor. Further, subsequent operational principles of the heart rate alarm system 200 are the same as those of the heart rate alarm system 100, so further description thereof is omitted for simplicity.

To sum up, the heart rate alarm system utilizes the heart rate measurement unit to measure a heart rate of the user to generate a heart rate value of the user through the at least two conductive units, and determines whether the heart rate value of the user is normal and generates a control signal according to a determination result. Then, the first transmission unit can transmit the control signal to the alarm device and transmit the heart rate value of the user to the second transmission unit. When the heart rate value of the user is abnormal, the alarm device can generate a flash and/or a special sound effect to notify other people to rescue the user. In addition, the operation unit can calculate a heart beat period, a heart physiological age, indicators of autonomic nervous system, and/or a calorie consumption indicator of the user according to the heart rate value received by the second transmission unit. Because the at least two conductive units are coupled to the clothing through at least two metal tenons, at least two metal buttons, at least two conductive velcros, and/or at least two magnets, or disposed at inner sides of at least two pieces of tarpaulin of the clothing, the at least two conductive units do not slip on the skin of the user after the user sweats. Thus, the present invention not only can provide a beautiful, comfortable and accurate heart rate measurement tool, but also can monitor the heart rate of the user to alarm the user any time and place.

## Claims

1. A heart rate alarm system (100, 200), comprising:
an article of clothing (102);
**characterized by**:
at least two conductive units (104, 106), **characterized in that** two conductive units (104, 106) of the at least two conductive units are two elastic bands, and the at least two conductive units (104, 106) are coupled to each other through at least one conductive fibric (118) of the clothing (102);
at least one alarm device (108) disposed on the clothing (102) for generating at least one alarm signal;
a heart rate measurement unit (110) coupled to the at least two conductive units (104, 106) for measuring a heart rate of a user to generate a heart rate value of the user through the at least two conductive units (104, 106), determining whether the heart rate value of the user is normal, and generating a control signal according to a determination result through the at least two conductive units (104, 106);
a first transmission unit (112) coupled to the heart rate measurement unit (110) for transmitting the control signal to the at least one alarm device (108), and transmitting the heart rate value;
a second transmission unit (114) for receiving the heart rate value of the user; and
an operation unit (116) coupled to the second transmission unit (114) for calculating a heart beat period, a heart physiological age, indicators of an autonomic nervous system, and/or a calorie consumption indicator of the user according to the heart rate value of the user.

2. The heart rate alarm system (100, 200) of claim 1, **characterized in that** the clothing (102) is long-sleeved clothing or short-sleeved clothing.

3. The heart rate alarm system (100, 200) of claim 2, **characterized in that** the two elastic bands are connected to two cuffs of the clothing (102) through at least two metal tenons, at least two metal buttons, at least two conductive velcros, and/or at least two magnets.

4. The heart rate alarm system (100, 200) of claim 2, **characterized in that** the heart rate measurement unit (110) is connected to the clothing (102) through at least one metal tenon, at least one metal button, at least one conductive velcro, and/or at least one magnet.

5. The heart rate alarm system (100, 200) of claim 2, **characterized in that** the first transmission unit (112) is connected to the heart rate measurement unit (110) through at least one metal tenon, at least one metal button, at least one conductive velcro, and/or at least one magnet.

6. The heart rate alarm system (100, 200) of claim 1, **characterized in that** the two elastic bands comprise conductive fibrics and/or conductive adhesive.

7. The heart rate alarm system (100, 200) of claim 1, **characterized in that** the clothing (102) further comprises two pieces of tarpaulin (1024, 1026), and the two elastic bands are disposed at inner sides of the two pieces of tarpaulin (1024, 1026), respectively

8. The heart rate alarm system (100, 200) of claim 7, **characterized in that** the heart rate measurement unit (110) is disposed at an inner side of one piece of tarpaulin of the two pieces of tarpaulin (1024, 1026), and at least one passive component of the heart rate measurement unit (110) is composed of at least one conductive fibric of the clothing (102).

9. The heart rate alarm system (100, 200) of claim 8, **characterized in that** the first transmission unit (112) is disposed at an inner side of one piece of tarpaulin of the two pieces of tarpaulin (1024, 1026), and at least one passive component of the first transmission unit (112) is composed of at least one conductive fibric of the clothing (102).

10. The heart rate alarm system (100, 200) of claim 1, **characterized in that** the at least one alarm device (108) comprises a light alarm unit (1082) and/or a special sound effect unit, and the at least one alarm signal is a flash and/or a special sound effect.

11. The heart rate alarm system (100, 200) of claim 1, **characterized in that** the first transmission unit (112) communicates with the at least one alarm device (108) through a Wireless Local Area Network (WLAN), a Zigbee (IEEE 802.15.4), a Bluetooth, a Bluetooth Low Energy (BLE), a Worldwide Interoperability for Microwave Access (WiMAX), a Global System for Mobile Communications (GSM), a General Packet Radio Service (GPRS), a Third Generation (3G), or an Actor Network Theory+ (Ant+) technology.

12. The heart rate alarm system (100, 200) of claim 1, **characterized in that** the second transmission unit (114) and the operation unit (116) are located outside the clothing (102), and the second transmission unit (114) further uploads the heart beat period, the heart physiological age, the indicators of the autonomic nervous system, and/or the calorie consumption indicator of the user to a cloud platform of the Internet.

13. The heart rate alarm system (100, 200) of claim 1, **characterized in that** the first transmission unit (112) communicates with the second transmission unit (114) through a transmission line.

14. The heart rate alarm system (100, 200) of claim 1, **characterized in that** the first transmission unit (112) communicates with the second transmission unit (114) through a Wireless Local Area Network, a Zigbee, a Bluetooth, a Bluetooth Low Energy, a Worldwide Interoperability for Microwave Access, a Global System for Mobile Communications, a General Packet Radio Service, a Third Generation, or an Actor Network Theory+ technology.
